# EUROPÄISCHE PATENTSCHRIFT

(11) **EP 0 508 511 B1**
(45) Veröffentlichungstag und Bekanntmachung des Hinweises auf die Patenterteilung: **10.12.1997**
(21) Anmeldenummer: 92200728.1
(22) Anmeldetag: 07.03.1992
(51) Int. Cl.: A61K 38/55

(54) **Erzeugnisse, enthaltend Verapamil und Trandolapril**
Compositions containing verapamil and trandolapril
Compositions contenant du verapamil et du trandolapril

(30) Priorität: 20.03.1991 DE 4109134
(43) Veröffentlichungstag der Anmeldung: 14.10.1992
(73) Patentinhaber: KNOLL AG, D-67061 Ludwigshafen (DE)
(72) Erfinder: Eckhardt, Albin, Dr., W-6901 Wilhelmsfeld (DE); Gries, Jozef, Dr., W-6706 Wachenheim (DE); Kirsten, Edward B., Nutley, N.J. 07110 (US); Lehmann, Hans Dieter, Prof. Dr., W-6945 Hirschberg (DE)
(74) Vertreter: Karau, Wolfgang, Dr.

(56) Entgegenhaltungen:
- EP-A- 0 265 685
- EP-A- 0 288 732
- LA REVUE DE MEDICINE INTERNE, Bd. 7, Nr. 4, 1986, Seiten 433-440; A. SIMON: 'Hypertension artérielle essentielle: stratégie d'emploi des médicaments antihypertenseurs'

## Beschreibung

Es ist bereits bekannt, daß der Ca-Antagonist Verapamil (Merck-Index 1989, Nr. 9851) und der ACE-Inhibitor Trandolapril (= N-(1s-Carbethoxy-3-phenylpropyl)-S-alanyl-2S,3aR,7aS-octa-hydroindol-2-carbonsäure, US-PS 4,933,361) blutdrucksenkende Eigenschaften besitzen.

Ebenso sind Kombinationen von Verapamil mit anderen ACE-Inhibitoren (EP 288 732) und von Trandolapril mit anderen Ca-Antagonisten (EP 265 685) beschrieben.

Die vorliegende Erfindung betrifft Arzneimittel enthaltend Verapamil und Trandolapril im Verhältnis 500:1 bis 10:1.

Das angegebene Verhältnis bezieht sich auf Gewichtsteile. Bevorzugt ist ein Verhältnis von 300:1 bis 50:1.

In der Kombination kann das Verapamil in Form eines physiologisch verträglichen Salzes vorliegen. Zur Salzbildung mit Verapamil kommen insbesondere Salzsäure, Schwefelsäure, Phosporsäure, Essigsäure, Malonsäure, Bernsteinsäure, Fumarsäure, Maleinsäure, Zitronensäure, Weinsäure, Milchsäure, Amidosulfonsäure und Oxalsäure in Betracht. Bevorzugtes Satz ist das Hydrochlorid.

Das Trandolapril kann in der Kombination ebenfalls als Satz vorliegen. Da Trandolapril sowohl eine saure als auch basische Gruppe besitzt, kann es sowohl mit den oben genannten Säuren als auch mit physiologisch verträglichen Basen, wie Alkali- oder Erdalkalihydroxiden, Salze bilden. Bevorzugt ist das freie Trandolapril.

Die neue Kombination eignet sich zur Behandlung von Hypertonie, koronarer Herzkrankheit, Herzinsuffizienz, Herzrhythmusstörungen sowie zum Schutz der Nieren.

Die erfindungsgemäße Kombination kann in üblicher Weise oral verabfolgt werden.

Die Dosierung hängt von Alter, Zustand und Gewicht des Patienten sowie von der Applikationsart ab. In der Regel beträgt die tägliche Dosis zwischen 50 und 300 mg Verapamil und 0,2 bis 4 mg Trandolapril.

Die neue Kombination kann in den gebräuchlichen galenischen Applikationsformen fest oder flüssig angewendet werden, z.B. als Tabletten, Filmtabletten, Retardtabletten, Kapseln, Pulver, Granulate, Dragees, Pellets, Retardpellets oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln und/oder Antioxidantien verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 10 bis 90 Gew.-%.

In der Kombination können sowohl Verapamil alleine als auch beide Einzelstoffe in retardierter Form vorliegen. Das Verapamil liegt vorzugsweise als Retardform und das Trandolapril in einer Instant-Release-Form vor.

In pharmakologischen Untersuchungen wurde gefunden, daß weder Verapamil-SR allein (10 mg/kg/Tag) noch Trandolapril allein (3 mg/kg/Tag) den diastolischen Blutdruck wacher normotoner Hunde zu senken vermögen.

In Kombination bewirkten die genannten Dosen (10 mg/kg/Tag Verapamil-SR + 3,0 mg/kg/Tag Trandolapril) dagegen eine deutliche (14 bis 17 mm Hg), signifikante und anhaltende (> 24 h) Senkung des diastolischen Blutdruckes.

Weiterhin wurde gefunden, daß bei diesen Tieren die Behandlung mit Verapamil-SR allein eine Verlängerung der atrio-ventrikulären überleitungszeit (PR-Zeit) und bei einem Teil der Tiere AV-Blockierung II. Grades zur Folge hatte. Diese Nebenwirkungen waren unter der Gabe der Kombination nicht stärker ausgeprägt.

In Versuchen an Hochdruckratten senkte die Kombination den Blutdruck nach wiederholter oraler Applikation signifikant stärker, als nach der Summe aus den Wirkungen der Einzelsubstanzen zu erwarten war.

Aus diesen Ergebnissen kann ebenfalls gefolgert werden, daß die erwünschte Blutdrucksenkung bei Gabe der Kombination verstärkt ist, die Nebenwirkung auf die atrio-ventrikuläre Überleitung jedoch nicht. Daraus resultiert eine Vergrößerung der therapeutischen Breite.

Diese an Tieren gefundene überadditive Wirkung der Kombination zeigt sich auch an Patienten mit Bluthochdruck.

Darüber hinaus wurde überraschend gefunden, daß Trandolapril die Bioverfügbarkeit von Verapamil ganz wesentlich erhöht.

### Beispiel

In eine Hartgelatinekapsel wurden eine Verapamil-HCl-Retardtablette mit 120 mg Wirkstoff und ein Trandolapril-Granulat mit 0,5 mg nicht reardiertem Trandolapril gefüllt und anschließend die Kapsel verschlossen.

## Patentansprüche

1. Arzneimittel, enthaltend Verapamil und Trandolapril im Verhältnis 500:1 bis 10:1.

2. Arzneimittel gemäß Anspruch 1, dadurch gekennzeichnet, daß das Verapamil in einer Retardform vorliegt.

3. Verfahren zur Herstellung eines Arzneimittels, dadurch gekennzeichnet, daß man Verapamil und Trandolapril zusammen mit in der Galenik üblichen Hilfsstoffen zu einer galenischen Form verarbeitet.

## Claims

1. A drug containing verapamil and trandolapril in the ratio from 500:1 to 10:1.

2. A drug as claimed in claim 1, wherein verapamil is present in a slow release form.

3. A process for preparing a drug, which comprises processing verapamil and trandolapril together with conventional pharmaceutical auxiliaries to give a pharmaceutical form.

## Revendications

1. Médicament contenant du vérapamil et du trandolapril dans le rapport de 500:1 à 10:1.

2. Médicament suivant la revendication 1, caractérisé en ce que le vérapamil se présente sous forme retard.

3. Procédé de préparation d'un médicament, caractérisé en ce que l'on convertit le vérapamil et le trandolapril en commun avec des adjuvants galéniques usuels sous une forme galénique.
